# EUROPEAN PATENT SPECIFICATION

(11) **EP 4 106 633 B1**
(45) Date of publication and mention of the grant of the patent: **23.08.2023**
(21) Application number: 21702675.6
(22) Date of filing: 03.02.2021
(51) Int. Cl.: A61B 8/08

(54) **METHODS AND SYSTEMS FOR FETAL HEART ASSESSMENT**
VERFAHREN UND SYSTEME ZUR FÖTALEN HERZBEURTEILUNG
PROCÉDÉS ET SYSTÈMES D'ÉVALUATION DU COEUR DU FOETUS

(30) Priority: 20.02.2020 US 202062978991 P; 02.04.2020 EP 20167678
(43) Date of publication of application: 28.12.2022
(73) Proprietor: Koninklijke Philips N.V., 5656 AG Eindhoven (NL)
(72) Inventor: KRISHNAN, Karthik, 5656 AE Eindhoven (NL); KUMAR, Karan, 5656 AE Eindhoven (NL); FIRTION, Celine, 5656 AE Eindhoven (NL); CHATTERJEE, Saunak, 5656 AE Eindhoven (NL); VAJINEPALLI, Pallavi, 5656 AE Eindhoven (NL); TRAHMS, Robert, Gustav, 5656 AE Eindhoven (NL); CANFIELD, Earl, M., 5656 AE Eindhoven (NL); RIELLY, Matthew, 5656AE Eindhoven (NL)
(74) Representative: Philips Intellectual Property & Standards
(86) International application number: PCT/EP2021/052462
(87) International publication number: WO 2021/165037

(56) References cited:
- EP-A1- 3 513 731
- EP-A1- 3 590 436
- EP-A2- 2 735 272
- WO-A1-2014/039935
- LAMI YEO ET AL: "Fetal Intelligent Navigation Echocardiography (FINE): a novel method for rapid, simple, and automatic examination of the fetal heart", ULTRASOUND IN OBSTETRICS AND GYNECOLOGY, vol. 42, no. 3, 2 September 2013 (2013-09-02), pages 268-284, XP055171261, ISSN: 0960-7692, DOI: 10.1002/uog.12563

## Description

### FIELD OF THE INVENTION

This invention relates to the field of fetal ultrasound imaging, and more specifically to the field of fetal heart ultrasound assessment.

### BACKGROUND OF THE INVENTION

Congenital heart disease (CHD) is a common condition that affects roughly 1% of live births and represents about 1/3 of all congenital diseases. Fetal ultrasound screening is recommended for every pregnant woman worldwide between 18-24 weeks of gestation and provides at least 5, and up to 8, recommended screening views of the fetal heart. The fetal heart is a complex structure and to screen for anomalies, the recommended views involve both B-mode (grayscale), Doppler (color) and M-mode ultrasound to understand the structure and function of the fetal heart.

CHD can be asymptomatic in fetal life but cause significant morbidity and mortality after birth and represents the leading cause of infant death in neonates born with birth defects. The earlier CHD is diagnosed, the better the outcomes and therapeutic options at birth. There are also increasingly available and effective in-utero therapies for specific CHD lesions (such as in-utero aortic valvuloplasty for HLHS) which can significantly improve the health of the fetus. These potential benefits all rely on accurate antenatal diagnosis of CHD. The antenatal diagnosis rate for CHD in the community is 30-50%, even in regions where antenatal ultrasound is universal.

The primary reason for this diagnosis gap is inadequate expertise in interpreting fetal cardiac images, due to the diagnostic challenge presented by a small and fast-beating fetal heart and a relatively low exposure to congenital heart disease among caregivers. Signs of cardiac disease are often subtle, necessitating careful targeted examination. Evaluation of the fetal heart is routinely performed at 18 and 22 weeks gestational age, although some forms of congenital heart disease may even be recognized during earlier stages of pregnancy and other CHDs may appear or be detected later.

There is therefore a need for a means of more accurately identifying and assessing CHD in an antenatal ultrasound investigation.

EP3590436A1 discloses a method of identifying, from a number of ultrasound images acquired during an ultrasound examination of a subject, the ultrasound image best suited to analyze a predetermined anatomical structure of the subject. In accordance with the method described therein, an image similarity index between the ultrasound image and a reference image is calculated for each of the number of ultrasound images. Next, for at least the ultrasound image(s) having the best image similarity index, a biometric parameter from the ultrasound image is calculated, and a biometric similarity index between the biometric parameter as determined from the ultrasound image and a corresponding biometric parameter derived from a physical examination is calculated. The optimal ultrasound image is then selected based on the biometric similarity index.

### SUMMARY OF THE INVENTION

The invention is defined by the claims.

According to examples in accordance with an aspect of the invention, there is provided a method for deriving a biometric parameter of a fetal heart, the method comprising:
acquiring a plurality of ultrasound images of a region of interest, wherein the region of interest comprises a fetal heart;
comparing the plurality of ultrasound images to a predefined clinical view;
selecting a group of ultrasound images related to the predefined clinical view based on the comparison, wherein the group of ultrasound images represents at least one cardiac cycle;
detecting an anatomical landmark of the fetal heart within an ultrasound image of the group of ultrasound images;
detecting or tracking the anatomical landmark of the fetal heart across the group of ultrasound images; and
deriving a biometric parameter of the fetal heart based on the detected or tracked anatomical landmark from one or more ultrasound images of the group of ultrasound images.

The method provides for a means of accurately assessing a biometric parameter(s) of a fetal heart.

The fetal heart is typically difficult to investigate due to its motion and high variability.

By automatically selecting a group of ultrasound images corresponding to a desired clinical view over a complete cardiac cycle and tracking an anatomical landmark across said group of images, it is possible to accurately derive biometric parameters of the fetal heart.

In an embodiment, tracking the anatomical landmark comprises:
automatically detecting an anatomical landmark in an ultrasound image with a dynamic model of the fetal heart; and
detecting and tracking the anatomical landmark across the group of ultrasound images with the dynamic motion model of the fetal heart.

In this way, the anatomical landmark may be tracked with point precision, thereby increasing the accuracy of the derived biometric parameter.

In a further embodiment, the plurality of ultrasound images comprises:
2D ultrasound images, wherein the tracking point of the dynamic motion model is derived from an image; or
3D ultrasound images, wherein the tracking point of the dynamic motion model is derived from a volume.

In an embodiment, the method comprises detecting a plurality of anatomical landmarks, tracking the plurality of anatomical landmarks across the group of ultrasound images and deriving a biometric parameter of the fetal heart based on the plurality of tracked anatomical landmarks.

By tracking a plurality of anatomical landmarks, a greater variety of biometric parameters may be derived with greater accuracy.

In an embodiment, the plurality of ultrasound images are 2D ultrasound images, and wherein the method comprises deriving a 3D biometric parameter based on the plurality of tracked anatomical landmarks.

In this way, a 3D biometric parameter, such as cardiac chamber volume, may be accurately derived from 2D ultrasound images.

In an embodiment, the predefined clinical view comprises one or more of:
an abdominal view;
a four chamber view;
a left ventricular outflow tract view;
a right ventricular outflow tract view;
a three vessel view;
a three vessel trachea view;
an aortic arch view; and
a ductal arch view.

In an embodiment, the predefined clinical view comprises a plurality of views.

In a further embodiment, the method comprises bookmarking a 2D ultrasound image for each of the plurality of view at a common point in the cardiac cycle.

In this way, from a routine 2D ultrasound scan, the fetal heart may be viewed at the same point in the cardiac cycle from a plurality of different view planes.

In an embodiment, the method further comprises bookmarking an ultrasound image of interest based on the derived biometric parameter.

In this way, an image relevant to the biometric parameter may be bookmarked for reference.

In an embodiment, the method further comprises bookmarking a cineloop of ultrasound images of interest based on the derived biometric parameter.

In this way, a series of images (for instance one cardiac cycle of a given view such as a Four Chamber view or all the relevant views in a cardiac cycle) relevant to the biometric parameter may be bookmarked for reference.

In an embodiment, the biometric parameter is derived from one or more of:
an abdominal parameter;
a thoracic parameter;
an atrial parameter;
a ventricular parameter;
an arterial parameter;
a wall parameter and
a valve parameter.

In an embodiment, the method further comprises:
identifying a gate location within an ultrasound image of the group of ultrasound images for placing a Doppler gate;
tracking the gate location across the group of ultrasound images based on the anatomical landmark; and
automatically positioning the Doppler gate at the tracked gate location.

In this way, color Doppler data may be automatically acquired for the fetal heart. For example, the valves or septa of the heart may be tracked across the group of images in order to ensure the Doppler gate remains within the correct valves or septa. For instance one may place a gate on the IntraVentricular Septum to evaluate of blood flows directly between the left and right ventricles due to a hole in the septum.

In an embodiment, the method further comprises performing automatic M-mode data collection from the group of ultrasound images, wherein performing automatic M-mode data collection comprises:
defining one or more sets of beam lines on the group of ultrasound images automatically based on a tracked anatomical structure;
collecting M-mode data along the beam lines; and
tracking the beam lines at anatomical locations based on the tracked anatomical feature across the cardiac cycle.

In this way, precise data relating the movement along a given line at given anatomical locations may be obtained for further analysis. The M-mode data can for instance identify arrhythmias.

According to examples in accordance with an aspect of the invention, there is provided a computer program comprising computer program code means which is adapted, when said computer program is run on a computer, to implement the methods described above.

According to examples in accordance with an aspect of the invention, there is provided a system for deriving a biometric parameter of a fetal heart, the system comprising a processor is adapted to:
acquire a plurality of ultrasound images of a region of interest, wherein the region of interest comprises a fetal heart;
compare the plurality of ultrasound images to a predefined clinical view;
select a group of ultrasound images related to the predefined clinical view based on the comparison, wherein the group of ultrasound images represents at least one cardiac cycle;
detect an anatomical landmark of the fetal heart within the group of ultrasound images;
track the anatomical landmark of the fetal heart across the group of ultrasound images; and
derive a biometric parameter of the fetal heart based on the tracked anatomical landmark.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Figure 1 shows an ultrasound diagnostic imaging system to explain the general operation;
Figure 2 shows a method of the invention; and
Figure 3 shows a first ultrasound image of a fetal heart at end systole and a second ultrasound image at end diastole.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for deriving a biometric parameter of a fetal heart. The method includes acquiring a plurality of ultrasound images of a region of interest, wherein the region of interest comprises a fetal heart and comparing the plurality of ultrasound images to a predefined clinical view. A group of ultrasound images related to the predefined clinical view are selected based on the comparison, wherein the group of ultrasound images represents at least one cardiac cycle.

An anatomical landmark of the fetal heart is detected within an ultrasound image of the group of ultrasound images and the anatomical landmark of the fetal heart is detected or tracked across the group of ultrasound images. A biometric parameter of the fetal heart is then determined based on the detected or tracked anatomical landmark from one or more ultrasound images of the group of ultrasound images.

A variety of sonographic measurements, such as Biparietal Diameter (BPD), Crown-Rump Length (CRL), Head Circumference (HC), Abdominal Circumference (AC) and Femur Length (FL) may be used to estimate the Gestational Age (GA) of a fetus. These measures are all based on physical growth (mass or proportions), which is affected by genetic variations (e.g., head size and shape in fetuses), gender and inherent variability in the fetal growth process. The fetal heart rate and the fetal cardiac valve intervals derived from a fetal echocardiogram may be used as alternative measures to estimate GA and are shown to be correlated with CRL.

The fetal echocardiogram is a detailed evaluation of cardiac structure and function, typically involving a sequential segmental analysis of the fetal heart using up to 8 recommended views. The recommended views may include: (a) Abdominal view (ABDO); (b) Four chamber view (4C); (c) Left Ventricular Outflow Tract (LVOT); (d) Right Ventricular Outflow Tract (RVOT); (e) Three Vessel View (3VV); (f) Three Vessel Trachea View (3VT); (g) Aortic Arch (AA); and (h) Ductal Arch (DA). The segmental analysis includes an initial assessment of fetal right/left orientation and may be followed by an assessment of the following segments and their relationships: a visceral/abdominal situs, including stomach position and cardiac apex position; atrial assessment, including situs, systemic and pulmonary venous connections, venous anatomy, atrial anatomy (including septum) and foramen ovale; ventricular assessment, including position, atrial connections, ventricular anatomy (including septum), relative and absolute size, function and pericardium; great artery assessment (aorta, main and branch pulmonary arteries, and ductus arteriosus), including position relative to the trachea, ventricular connections and vessel size, patency, and flow (both velocity and direction); atrioventricular junction, including anatomy, size and function of atrioventricular (e.g., mitral and tricuspid) valves; and ventriculoarterial junction assessment, including anatomy, size, and function of semilunar (e.g., aortic and pulmonary) valves, including assessment of both the subpulmonary and subaortic regions.

However, the fetal heart is a difficult organ to measure using typical echocardiographic techniques.

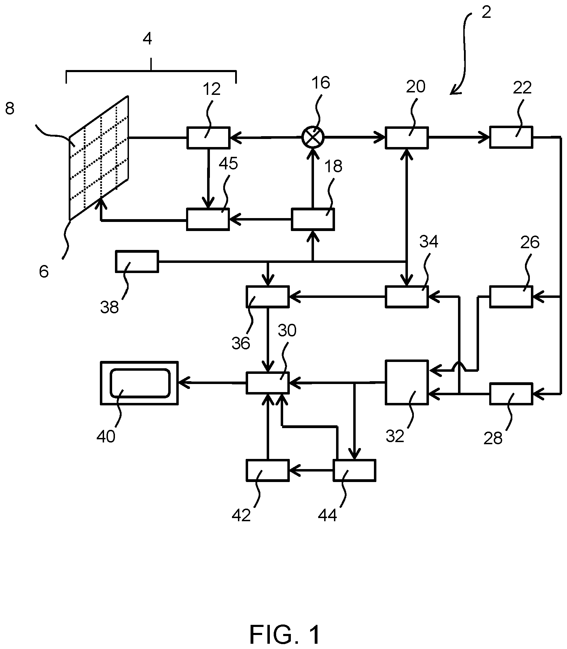

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

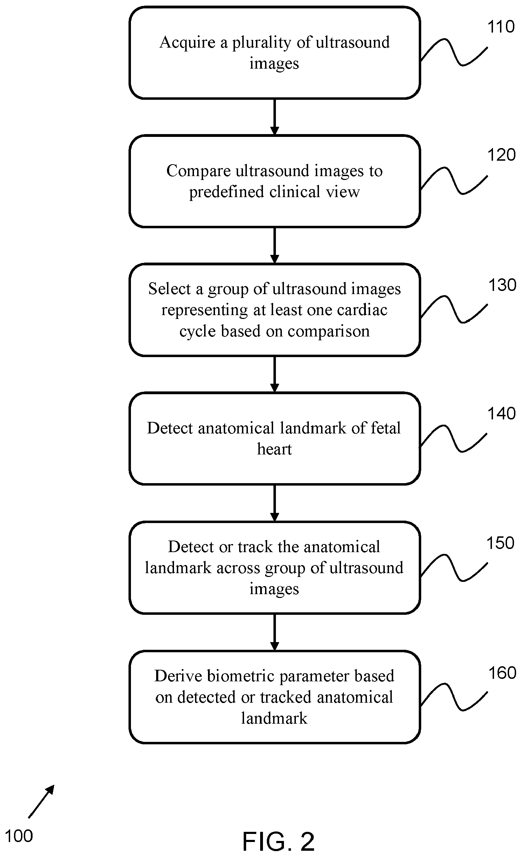

Figure 2 shows a method 100 a method for deriving a biometric parameter of a fetal heart.

The method begins in step 110 by acquiring a plurality of ultrasound images of a region of interest, wherein the region of interest comprises a fetal heart. The ultrasound images may comprise 2D ultrasound images and/or 3D ultrasound images.

In step 120, the plurality of ultrasound images is compared to a predefined clinical view. As described above, there are a variety of different clinical views that are taken into account in a typical fetal echocardiogram. For example, the predefined clinical view may comprise one or more of: an abdominal view; a four chamber view; a left ventricular outflow tract view; a right ventricular outflow tract view; a three vessel view; a three vessel trachea view; an aortic arch view; and a ductal arch view.

In step 130, a group of ultrasound images related to the predefined clinical view is selected based on the comparison, wherein the group of ultrasound images represents at least one cardiac cycle.

The group of ultrasound images are selected to match the predefined clinical views as closely as possible. The group of ultrasound images are selected to cover at least one cardiac cycle of the fetal heart as certain predefined clinical views require the fetal heart to be at a given point in the cardiac cycle in order to be accurate. For example, an accurate Four Chamber view requires the valves of the heart to be closed.

In step 140, an anatomical landmark of the fetal heart is detected within an ultrasound image of the group of ultrasound images.

For example, the anatomical landmark may be identified based on a model of pose points (also referred to as key points in a 2D image) of the view of the fetal heart. The desired point in the cardiac cycle for a given clinical view may be selected from the group of ultrasound images by tracking the pose points in real time. The anatomical landmarks associated with the pose points may include, for example, aortic and ductal arches, inferior and superior vena cava, trachea and the like. Further, a plurality of anatomical landmarks may be taken into account and detected within the ultrasound image.

In step 150, the anatomical landmark of the fetal heart is detected or tracked across the group of ultrasound images.

Tracking the anatomical landmark, may be performed, for example, by automatically detecting an anatomical landmark in an ultrasound image with a dynamic model of the fetal heart and detecting and tracking the anatomical landmark across the group of ultrasound images with the dynamic motion model of the fetal heart.

In the example of a dynamic model, the model may estimate the cardiac cycle directly from B-mode ultrasound data and be used to detect conditions such as arrhythmia. The dynamic model may also be used to automatically place and track Doppler gates dynamically and accurately following the heart's motion to examine the valves, veins, arteries, septa and foramen ovale and hence potentially detect related anatomical and functional anomalies. The placement of Doppler gates is discussed further below.

Further, in the example of a dynamic model, the model may also detect functional anomalies such as dysfunctional valves, foramen ovale's membrane flapping in the wrong atrium and the like.

In step 160, a biometric parameter of the fetal heart is derived based on the detected or tracked anatomical landmark from one or more ultrasound images of the group of ultrasound images. The biometric parameter may be derived from one or more of: an abdominal parameter; a thoracic parameter; an atrial parameter; a ventricular parameter; an arterial parameter; a wall parameter and a valve parameter.

The method provides a means of generating a fingerprint of the fetal heart during the live scan. The fingerprint may be built from key anatomical landmarks detected in real time on the structure of the fetal heart from live ultrasound data. In this way, a model of the fetal heart may be built, from which several parameters such as biometrics, structural and functional anomalies can be detected. The model can be used to detect optimal scan planes and fetal screening views during routine ultrasound scanning of the fetal heart.

The method may operate on any ultrasound image. For example, the plurality of ultrasound images may comprise 2D ultrasound images, in which case a tracking point of the dynamic motion model is derived from a 2D image. In another example, the plurality of ultrasound images may comprise 3D ultrasound images, wherein a tracking point of the dynamic motion model is derived from a volume.

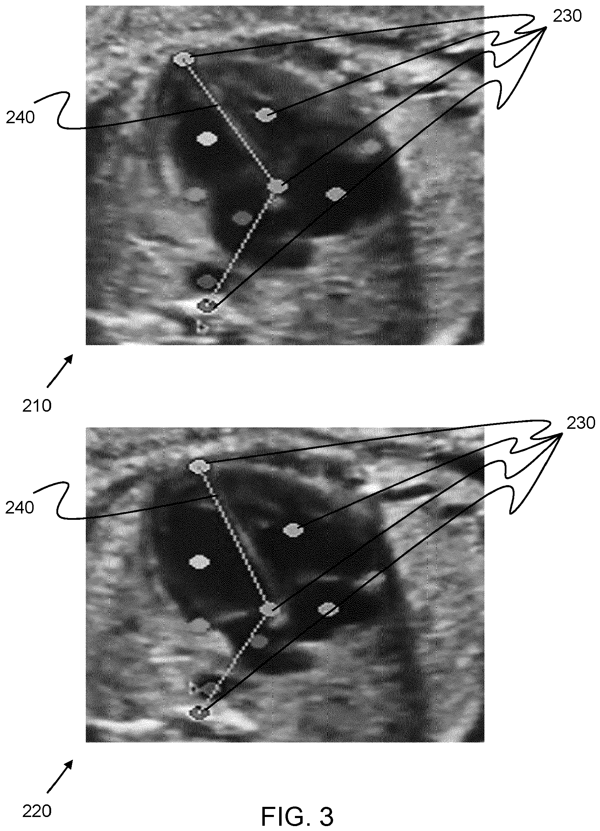

Figure 3 shows a first ultrasound image 210 of a fetal heart at end systole (end of the ejection phase) and a second ultrasound image 220 at end diastole (end of the ventricular filling).

The first and second ultrasound images comprise tracking points 230 corresponding to anatomical landmarks within the fetal heart according to an aspect of the invention. In the images shown in Figure 3, the tracking points 230 have been used to define a Cardiac Axis 240, which is commonly used to derive biometrics from a fetal heart.

The Cardiac Axis (CAx) on a normal four chamber fetal heart image is expected to be 45° ± 15°. The axis is defined as the line from the spine to the crux of the heart with the apex of the heart.

The nature of an abnormal CAx and the CAx shift within the fetal cardiac cycle depend on the type of CHD. Zhao et. al, Cardiac axis shift within the cardiac cycle of normal fetuses and fetuses with congenital heart defect, Ultrasound in Obstetrics and Gynecology 2014 found that at 18 to 26 weeks of gestation, the mean CAx in normal fetal hearts is 45.9 ± 8.5° at end systole and 38.3 ± 8.4° at end diastole *(P <* 0.001). The mean CAx in fetuses with CHD reported in the study was 53.4 ± 17.8° at end systole and 47.5 ± 17.3° at end diastole *(P <* 0.001), resulting in an average difference of 7.6 ± 3.2°. However, in some forms of CHD, such as hypoplastic left heart syndrome and L-transposition of the great arteries, the CAx was greater at end diastole than at end systole, with a difference of more than 5°.

In Hornberger et al, Re: Cardiac axis shift within the cardiac cycle of normal fetuses and fetuses with congenital heart defect, Ultrasound in Obstetrics and Gynecology 2015*,* the authors conclude that the cardiac axis demonstrated least variability among controls in end-systole and was more abnormal (abnormal being defined as < 25° or > 65°) in those with CHD in end systole, suggesting that end systole may be the best time in the cardiac cycle at which to assess the cardiac axis. The study concludes that the heart pivots or swings from diastolic phase to systolic phase. The authors study the unique mechanical properties of the myocardial and circumferential fibers of the ventricles and twist mechanics that is responsible for the swing and differentiate the contraction patterns of the hypoplastic left heart syndrome that results in this behavior.

The automatically tracked points 230 at the junctions of the valves, apex and aorta may be used to quantify the mechanics of the heart motion throughout the cardiac cycle leading to improved understanding of biomechanical anomalies in CHD.

In addition to the Cardiac Axis, the tracked points may be used to assess atrium and ventricle sizes. In particular, tracking points located at the left atrium, the right atrium, the mitral valve junction and the tricuspid valve junction can be used to provide the size of the left and right atria. Quantifying the atrium sizes of a fetal heart may be used, for example, to identify Ebstein's anomaly, which is characterized by an enlarged right atrium. In a similar manner, tracking points located at the left ventricle, the right ventricle, the mitral valve junction and the tricuspid valve junction may be used to predict the relative sizes of the left and right ventricles, which are expected to be approximately equal in size. The tracking points may be tracked at all relevant phases of the cardiac cycle.

By monitoring the size of the atria and ventricles across at least one cardiac cycle, it is possible to determine a measure for the function of the cardiac chambers, which may also contribute to the biometric parameter.

In some applications, 3D ultrasound imaging may not be possible; however, 3D biometric parameters may still be derived from 2D ultrasound images based on a plurality of tracked anatomical landmarks.

In routine clinical workflow, volumes may be measured from 2D ultrasound images using the Simpson rule, which assumes that the cross-section of each ventricular slice is cylindrical and that the total volume is the sum of all cylinders present in the image. These measurements may then be plotted against gestational age (as determined by measurement of biparietal diameter or fetal length). Normalized charts at various gestational ages exist for the right ventricle deft ventricle ratio, for example. The methods described above provide not just for automated estimates of these biometrics, but also more accurate 3D approximation of these biometrics across all phases of the cardiac cycle. This is possible because the points are tracked across all views and across all phases of the cardiac cycle leading to more accurate patient specific shape approximations.

Apart from the mitral and the tricuspid valve, the fetal circulatory system uses shunts to bypass the lungs and liver, that are not fully developed. These are the foramen ovale, which moves blood from the right atrium of the heart to the left atrium, and the ductus arteriosus, which moves blood from the pulmonary artery to the aorta. Newborns with CHD may also present with persistent foramen ovale and/or ductus arteriosus. By tracking these points in the obtained ultrasound images, an abnormal cardiac axis, enlarged right atrium, atrial septal defect, AV septal defect, truncus arteriosus, transposition of great arteries, Single ventricle, hypoplastic left ventricle, hypoplastic right heart syndrome, hypoplastic left heart syndrome, coarctation, valvular anomalies and the like, may be detected in the antenatal ultrasound images.

As the tracking points, valves, septa and junctions may be tracked in real time, the method may provide for automatic modes on a user interface to jump to the evaluation of any given anatomical landmark, such as the Foramen ovale or the mitral valve for instance, and provide automation therein to set the focal point / depth / preset parameters for evaluation by tracking these pose points. This may help a less experienced sonographer, or speed up exam time, for the fetal ultrasound screening.

The method may further comprise bookmarking an ultrasound image of interest based on the derived biometric parameter. For example, based on a derived biometric parameter, the user may wish to investigate said parameter further. By automatically bookmarking one or more images that form the basis of the derived biometric parameter, the user may better understand where the measurement arose from. In addition to still ultrasound image frames the method may further comprise bookmarking a cineloop of ultrasound images of interest based on the derived biometric parameter.

An ultrasound image may be bookmarked for a plurality of different clinical views at the same point in the cardiac cycle. By computing the relative distance between tracked points, a model may be used to identify consistent points in the cardiac cycle for bookmarks of different views.

The method may further comprise identifying a gate location within an ultrasound image of the group of ultrasound images for placing a Doppler gate, tracking the gate location across the group of ultrasound images based on the anatomical landmark and automatically positioning the Doppler gate at the tracked gate location.

Doppler evaluation of the fetal heart may be used to evaluate septal and valvular defects. The diastolic perfusion across the atrioventricular valves may be assessed by using color Doppler ultrasound measurements in an apical or basal approach. Pulsed Doppler ultrasound may be used to observe a typical biphasic shape of the diastolic flow velocity waveform with an early peak diastolic velocity and a second peak during atrial contraction. In this plane, regurgitation across the atrioventricular valves, which is more frequent at the tricuspid valve, nay be detected during systole with color Doppler imaging. Flow across the foramen ovale may be visualized in a lateral approach of the four-chamber view. Color Doppler imaging may provide for confirmation of the physiological right-to-left shunt and visualization of the pulmonary veins as they enter the left atrium.

The Doppler acquisitions described above require Doppler gates to be placed at relevant locations for each of the above assessments. The gate locations can be automatically detected using the tracked points described above. For example, the tracked points may be used for placing color Doppler windows on four chamber views. Furthermore, the tracked points may also be used to place pulsed Doppler gates on the valves to measure atrioventricular flow.

In addition, the method may further comprise performing automatic M-mode data collection from the group of ultrasound images. Automatic M-mode data collection may comprise defining one or more sets of beam lines on the group of ultrasound images automatically based on a tracked anatomical structure, collecting M-mode data along the beam lines and tracking the beam lines at anatomical locations based on the tracked anatomical feature across the cardiac cycle.

The dynamic model described above may be utilized for automated definition M-mode data collection, where the line is drawn between relevant selected structures, such as: atria; ventricles; left atrium with left ventricle; right atrium with right ventricle; and cross left/right. Combined with the anatomical intelligence of the model due to the known anatomical context of the tracking points, thee obtained data may therefore be used to detect arrhythmia and precisely identify where it is coming from.

The methods described above may be integrated into any suitable ultrasound imaging system, or other processing system, thereby providing a workflow guided evaluation of the heart for analysis of the fetal heart by identifying key anatomical landmarks and tracking them in real time to be able to evaluate structural and functional aspects of each of these structures in isolation. Such a system may, for example, include a user-interface for one click evaluation of structures of the fetal heart to identify fetal heart defects.

Put another way, fetal heart evaluation may be simplified and a guided workflow imposed.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method (100) for deriving a biometric parameter of a fetal heart, the method comprising:
acquiring (110) a plurality of ultrasound images of a region of interest, wherein the region of interest comprises a fetal heart;
comparing (120) the plurality of ultrasound images to a predefined clinical view;
selecting (130) a group of ultrasound images related to the predefined clinical view based on the comparison, wherein the group of ultrasound images represents at least one cardiac cycle;
detecting (140) an anatomical landmark of the fetal heart within an ultrasound image of the group of ultrasound images;
detecting (150) or tracking the anatomical landmark of the fetal heart across the group of ultrasound images; and
deriving (160) a biometric parameter of the fetal heart based on the detected or tracked anatomical landmark from one or more ultrasound images of the group of ultrasound images.

2. A method (100) as claimed in claim 1, wherein tracking the anatomical landmark comprises:
automatically detecting an anatomical landmark in an ultrasound image with a dynamic model of the fetal heart; and
detecting and tracking the anatomical landmark across the group of ultrasound images with the dynamic motion model of the fetal heart.

3. A method (100) as claimed in claim 2, wherein the plurality of ultrasound images comprises:
2D ultrasound images, wherein a tracking point of the dynamic motion model is derived from an image; or
3D ultrasound images, wherein the tracking point of the dynamic motion model is derived from a volume.

4. A method (100) as claimed in any of claims 1 to 3, wherein the method comprises detecting a plurality of anatomical landmarks, tracking the plurality of anatomical landmarks across the group of ultrasound images and deriving a biometric parameter of the fetal heart based on the plurality of tracked anatomical landmarks.

5. A method (100) as claimed in any of claims 1 to 4, wherein the plurality of ultrasound images are 2D ultrasound images, and wherein the method comprises deriving a 3D biometric parameter based on the plurality of tracked anatomical landmarks.

6. A method (100) as claimed in any of claims 1 to 5, wherein the predefined clinical view comprises one or more of:
an abdominal view;
a four chamber view;
a left ventricular outflow tract view;
a right ventricular outflow tract view;
a three vessel view;
a three vessel trachea view;
an aortic arch view; and
a ductal arch view.

7. A method (100) as claimed in any of claims 1 to 6, wherein the predefined clinical view comprises a plurality of views.

8. A method (100) as claimed in claim 7, wherein the method comprises bookmarking a 2D ultrasound image for each of the plurality of view at a common point in the cardiac cycle.

9. A method (100) as claimed in any of claims 1 to 8, wherein the method further comprises bookmarking an ultrasound image of interest based on the derived biometric parameter.

10. A method (100) as claimed in any of claims 1 to 9, wherein the method further comprises bookmarking a cineloop of ultrasound images of interest based on the derived biometric parameter.

11. A method (100) as claimed in any of claims 1 to 10, wherein the biometric parameter is derived from one or more of:
an abdominal parameter;
a thoracic parameter;
an atrial parameter;
a ventricular parameter;
an arterial parameter;
a wall parameter and
a valve parameter.

12. A method (100) as claimed in any of claims 1 to 11, wherein the method further comprises:
identifying a gate location within an ultrasound image of the group of ultrasound images for placing a Doppler gate;
tracking the gate location across the group of ultrasound images based on the anatomical landmark; and
automatically positioning the Doppler gate at the tracked gate location.

13. A method (100) as claimed in any of claims 1 to 12, wherein the method further comprises performing automatic M-mode data collection from the group of ultrasound images, wherein performing automatic M-mode data collection comprises:
defining one or more sets of beam lines on the group of ultrasound images automatically based on a tracked anatomical structure;
collecting M-mode data along the beam lines; and
tracking the beam lines at anatomical locations based on the tracked anatomical feature across the cardiac cycle.

14. A system for deriving a biometric parameter of a fetal heart, the system comprising a processor which is adapted to
acquire (110) a plurality of ultrasound images (210, 220) of a region of interest, wherein the region of interest comprises a fetal heart;
compare (120) the plurality of ultrasound images to a predefined clinical view;
select (130) a group of ultrasound images related to the predefined clinical view based on the comparison, wherein the group of ultrasound images represents at least one cardiac cycle;
detect (140) an anatomical landmark (230) of the fetal heart within the group of ultrasound images;
track (150) the anatomical landmark of the fetal heart across the group of ultrasound images; and
derive (160) a biometric parameter of the fetal heart based on the tracked anatomical landmark.

15. A computer program comprising computer program code means which is adapted, when said computer program is run on a processor of a system of claim 14, to implement the method of any of claims 1 to 13.

## Patentansprüche

1. Verfahren (100) zum Ableiten eines biometrischen Parameters eines Fötusherzens, das Verfahren umfassend:
Erfassen (110) einer Vielzahl von Ultraschallbildern eines Bereichs von Interesse, wobei der Bereich von Interesse ein Fötusherz umfasst;
Vergleichen (120) der Vielzahl von Ultraschallbildern mit einer vordefinierten klinischen Ansicht;
Auswählen (130) einer Gruppe von Ultraschallbildern, die sich auf die vordefinierte klinische Ansicht beziehen, basierend auf dem Vergleich, wobei die Gruppe von Ultraschallbildern mindestens einen Herzzyklus darstellt;
Erkennen (140) eines anatomischen Orientierungspunkts des Fötusherzens in einem Ultraschallbild der Gruppe von Ultraschallbildern;
Erfassen (150) oder Verfolgen des anatomischen Orientierungspunkts des Fötusherzens in der Gruppe von Ultraschallbildern; und
Ableiten (160) eines biometrischen Parameters des Fötusherzens basierend auf dem erfassten oder verfolgten anatomischen Orientierungspunkt aus einem oder mehreren Ultraschallbildern der Gruppe von Ultraschallbildern.

2. Verfahren (100) nach Anspruch 1, wobei das Verfolgen des anatomischen Orientierungspunkts Folgendes umfasst:
automatisches Erkennen eines anatomischen Orientierungspunkts in einem Ultraschallbild mit einem dynamischen Modell des Fötusherzens; und
Erkennen und Verfolgen des anatomischen Orientierungspunkts in der Gruppe der Ultraschallbilder mit dem dynamischen Bewegungsmodell des Fötusherzens.

3. Verfahren (100) nach Anspruch 2, wobei die Vielzahl von Ultraschallbildern Folgendes umfasst:
2D-Ultraschallbilder, wobei ein Verfolgungspunkt des dynamischen Bewegungsmodells aus einem Bild abgeleitet ist; oder
3D-Ultraschallbilder, wobei der Verfolgungspunkt des dynamischen Bewegungsmodells aus einem Volumen abgeleitet ist.

4. Verfahren (100) nach einem der Ansprüche 1 bis 3, wobei das Verfahren ein Erfassen einer Vielzahl anatomischer Orientierungspunkte, ein Verfolgen der Vielzahl anatomischer Orientierungspunkte über die Gruppe von Ultraschallbildern und ein Ableiten eines biometrischen Parameters des Fötusherzens basierend auf der Vielzahl verfolgter anatomischer Orientierungspunkte umfasst.

5. Verfahren (100) nach einem der Ansprüche 1 bis 4, wobei die Vielzahl von Ultraschallbildern 2D-Ultraschallbilder sind und wobei das Verfahren ein Ableiten eines biometrischen 3D-Parameters auf der Grundlage der Vielzahl verfolgter anatomischer Orientierungspunkte umfasst.

6. Verfahren (100) nach einem der Ansprüche 1 bis 5, wobei die vordefinierte klinische Ansicht eines oder mehrere von Folgenden umfasst:
eine Bauchansicht;
eine Vier-Kammer-Ansicht;
eine Ansicht des linken ventrikulären Ausflusstrakts;
eine Ansicht des rechtsventrikulären Ausflusstrakts;
eine Dreigefäß-Ansicht;
eine Dreigefäß-Trachea-Ansicht;
eine Ansicht des Aortenbogens; und
eine Ansicht des Duktusbogens.

7. Verfahren (100) nach einem der Ansprüche 1 bis 6, wobei die vordefinierte klinische Ansicht eine Vielzahl von Ansichten umfasst.

8. Verfahren (100) nach Anspruch 7, wobei das Verfahren ein Setzen von Lesezeichen für ein 2D-Ultraschallbild für jede die mehreren Ansichten an einem gemeinsamen Punkt im Herzzyklus umfasst.

9. Verfahren (100) nach einem der Ansprüche 1 bis 8, wobei das Verfahren ferner ein Setzen von Lesezeichen für ein Ultraschallbild von Interesse basierend auf dem abgeleiteten biometrischen Parameter umfasst.

10. Verfahren (100) nach einem der Ansprüche 1 bis 9, wobei das Verfahren ferner ein Setzen von Lesezeichen für eine Kinodarstellung von Ultraschallbildern von Interesse basierend auf dem abgeleiteten biometrischen Parameter umfasst.

11. Verfahren (100) nach einem der Ansprüche 1 bis 10, wobei der biometrische Parameter von einem oder mehreren der folgenden Parameter abgeleitet ist:
einem abdominalen Parameter;
einem thorakalen Parameter;
einem atrialen Parameter;
einem ventrikulären Parameter;
einem arteriellen Parameter;
einem Wandparameter und
einem Klappenparameter.

12. Verfahren (100) nach einem der Ansprüche 1 bis 11, wobei das Verfahren ferner Folgendes umfasst:
Identifizieren einer Gate-Position innerhalb eines Ultraschallbilds der Gruppe von Ultraschallbildern zum Platzieren eines Doppler-Gates;
Verfolgen der Torposition über die Gruppe von Ultraschallbildern basierend auf dem anatomischen Orientierungspunkt; und
automatisches Positionieren des Doppler-Gates an der verfolgten Stelle des Gates.

13. Verfahren (100) nach einem der Ansprüche 1 bis 12, wobei das Verfahren ferner ein Ausführen einer automatischen M-Mode-Datenerfassung aus der Gruppe von Ultraschallbildern umfasst, wobei ein Ausführen der automatischen M-Mode-Datenerfassung Folgendes umfasst:
automatisches definieren eines oder mehrerer Sätze von Strahlenlinien basierend auf Ultraschallbildern basierend auf einer verfolgten anatomischen Struktur;
Sammeln von M-Mode-Daten entlang der Strahlenlinien; und
verfolgen der Strahlenlinien an anatomischen Stellen basierend auf dem verfolgten anatomischen Merkmal während des Herzzyklus.

14. System zum Ableiten eines biometrischen Parameters eines Fötusherzens, das System umfassend einen Prozessor, der zu Folgendem angepasst ist:
Erfassen (110) einer Vielzahl von Ultraschallbildern (210, 220) eines Bereichs von Interesse, wobei der Bereich von Interesse ein Fötusherz umfasst;
Vergleichen (120) der Vielzahl von Ultraschallbildern mit einer vordefinierten klinischen Ansicht; Auswählen (130) einer Gruppe von Ultraschallbildern, die sich auf die vordefinierte klinische Ansicht beziehen, basierend auf dem Vergleich, wobei die Gruppe von Ultraschallbildern mindestens einen Herzzyklus darstellt;
Erkennen (140) eines anatomischen Orientierungspunkts (230) des Fötusherzens innerhalb der Gruppe von Ultraschallbildern;
Verfolgen (150) des anatomischen Orientierungspunkts des Fötusherzens in der Gruppe von Ultraschallbildern; und
Ableiten (160) eines biometrischen Parameters des Fötusherzens basierend auf dem verfolgten anatomischen Orientierungspunkt.

15. Computerprogramm, umfassend Computerprogrammcode, der angepasst ist, um, wenn das Computerprogramm auf einem Prozessor eines Systems nach Anspruch 14 ausgeführt wird, das Verfahren nach einem der Ansprüche 1 bis 13 zu implementieren.

## Revendications

1. Méthode (100) de dérivation d'un paramètre biométrique d'un coeur de foetus, comprenant:
l'acquisition (110) d'une pluralité d'images ultrasonores d'une région d'intérêt, la région d'intérêt comprenant un coeur de foetus;
comparer (120) la pluralité d'images échographiques à une vue clinique prédéfinie;
sélectionner (130) un groupe d'images ultrasonores liées à la vue clinique prédéfinie sur la base de la comparaison, le groupe d'images ultrasonores représentant au moins un cycle cardiaque;
détecter (140) un repère anatomique du coeur du foetus dans une image échographique du groupe d'images échographiques;
détecter (150) ou suivre le repère anatomique du coeur du foetus dans le groupe d'images échographiques; et
déduire (160) un paramètre biométrique du coeur du foetus sur la base du repère anatomique détecté ou suivi à partir d'une ou plusieurs images échographiques du groupe d'images échographiques.

2. Méthode (100) selon la revendication 1, dans laquelle le suivi du repère anatomique consiste à:
détecter automatiquement un repère anatomique dans une image échographique à l'aide d'un modèle dynamique du coeur du foetus; et
détecter et suivre le repère anatomique dans le groupe d'images échographiques à l'aide du modèle de mouvement dynamique du coeur du foetus.

3. Méthode (100) selon la revendication 2, dans laquelle la pluralité d'images ultrasonores comprend:
des images échographiques 2D, dans lesquelles un point de suivi du modèle de mouvement dynamique est dérivé d'une image; ou
des images échographiques 3D, dans lesquelles le point de suivi du modèle de mouvement dynamique est dérivé d'un volume.

4. Méthode (100) selon l'une des revendications 1 à 3, dans laquelle la méthode comprend la détection d'une pluralité de repères anatomiques, le suivi de la pluralité de repères anatomiques dans le groupe d'images échographiques et la dérivation d'un paramètre biométrique du coeur du foetus sur la base de la pluralité de repères anatomiques suivis.

5. Procédé (100) selon l'une des revendications 1 à 4, dans lequel la pluralité d'images échographiques sont des images échographiques 2D, et dans lequel le procédé consiste à dériver un paramètre biométrique 3D sur la base de la pluralité de repères anatomiques suivis.

6. Méthode (100) selon l'une des revendications 1 à 5, dans laquelle la vue clinique prédéfinie comprend un ou plusieurs des éléments suivants:
une vue abdominale;
une vue à quatre chambres;
une vue de la voie d'écoulement du ventricule gauche;
une vue de la voie d'écoulement du ventricule droit;
une vision à trois navires;
une vue de la trachée à trois vaisseaux;
une vue de la crosse de l'aorte; et
une vue de l'arc canalaire.

7. Méthode (100) selon l'une des revendications 1 à 6, dans laquelle la vue clinique prédéfinie comprend plusieurs vues.

8. Méthode (100) selon la revendication 7, dans laquelle la méthode comprend l'enregistrement d'une image ultrasonore 2D pour chacune de la pluralité de vues à un point commun du cycle cardiaque.

9. Procédé (100) selon l'une des revendications 1 à 8, dans lequel le procédé comprend en outre la mise en signet d'une image ultrasonore d'intérêt sur la base du paramètre biométrique dérivé.

10. Procédé (100) selon l'une des revendications 1 à 9, dans lequel le procédé comprend en outre la mise en signet d'une boucle cinématique d'images ultrasonores d'intérêt sur la base du paramètre biométrique dérivé.

11. Méthode (100) selon l'une des revendications 1 à 10, dans laquelle le paramètre biométrique est dérivé d'un ou de plusieurs des éléments suivants:
un paramètre abdominal;
un paramètre thoracique;
un paramètre auriculaire;
un paramètre ventriculaire;
un paramètre artériel;
un paramètre de paroi et
un paramètre de la vanne.

12. Méthode (100) selon l'une des revendications 1 à 11, dans laquelle la méthode consiste en outre à:
identifier l'emplacement d'une porte dans une image ultrasonore du groupe d'images ultrasonores pour placer une porte Doppler;
suivre l'emplacement de la porte dans le groupe d'images échographiques sur la base du repère anatomique; et
positionner automatiquement la porte Doppler à l'emplacement de la porte suivie.

13. Méthode (100) selon l'une des revendications 1 à 12, dans laquelle la méthode comprend en outre la collecte automatique de données en mode M à partir du groupe d'images échographiques, dans laquelle la collecte automatique de données en mode M consiste à:
définir automatiquement un ou plusieurs ensembles de lignes de faisceau sur le groupe d'images ultrasonores en fonction d'une structure anatomique suivie;
recueillir des données en mode M le long des lignes de faisceaux; et
suivre les lignes de faisceaux à des emplacements anatomiques sur la base de la caractéristique anatomique suivie tout au long du cycle cardiaque.

14. Système de dérivation d'un paramètre biométrique d'un coeur de foetus, le système comprenant un processeur adapté pour:
acquérir (110) une pluralité d'images échographiques (210, 220) d'une région d'intérêt, dans laquelle la région d'intérêt comprend un coeur de foetus;
comparer (120) la pluralité d'images échographiques à une vue clinique prédéfinie;
sélectionner (130) un groupe d'images échographiques liées à la vue clinique prédéfinie, où le groupe d'images ultrasonores représentant au moins un cycle cardiaque;
détecter (140) un repère anatomique (230) du coeur du foetus dans le groupe d'images échographiques;
suivre (150) le repère anatomique du coeur du foetus dans le groupe d'images échographiques; et
dériver (160) un paramètre biométrique du coeur du foetus sur la base du repère anatomique suivi.

15. Programme d'ordinateur comprenant un moyen de code de programme d'ordinateur qui est adapté, lorsque ledit programme d'ordinateur est exécuté sur un processeur d'un système de la revendication 14, pour mettre en œuvre la méthode de l'une des revendications 1 à 13.
